# EUROPEAN PATENT SPECIFICATION

(11) **EP 1 214 021 B1**
(45) Date of publication and mention of the grant of the patent: **17.10.2007**
(21) Application number: 00991996.0
(22) Date of filing: 25.09.2000
(51) Int. Cl.: A61F 2/06

(54) **PRE-SHAPED INTRALUMINAL GRAFT**
VORGEFORMTES INTRALUMINALES TRANSPLANTAT
GREFFE INTRALUMINALE PREFORMEE

(30) Priority: 23.09.1999 AU PQ302999
(43) Date of publication of application: 19.06.2002
(73) Proprietor: Edwards Lifesciences Corporation, Irvine, CA 92625 (US); Endogad Research PTY Limited, Camperdown, NSW 2050 (AU)
(72) Inventor: DEHDASHTIAN, Mark, Costa Mesa, CA 92626 (US); JIMINEZ, Theodoro, Irvine, CA 92614 (US); WHITE, Geoffrey, H., East Balmain, NSW 2041 (AU); YU, Weiyun, Birchgrove, NSW 2041 (AU)
(74) Representative: Baker, Colin John
(86) International application number: PCT/US2000/026239
(87) International publication number: WO 2001/030270

(56) References cited:
- EP-A- 0 808 614
- WO-A-95/09585
- WO-A-99/01073
- WO-A-99/15102
- WO-A-99/32050
- WO-A-99/37242
- US-A- 5 904 714

## Description

### Field of the Invention

The present invention relates to an intraluminal device for use in the treatment of aneurysmal or stenotic disease. Particularly, the present invention relates to endovascular emplacement of structures designed to enhance a patient's vasculature, for example through the extension of ostensively aneurysmal growths, dissections or related issues.

### Background of the Invention

Endovascular grafts and stented grafts are generally known to be useful in several distinct configurations. For example, it is known to use intraluminal grafts and stents of various designs for the treatment of aneurysms such as aortic aneurysms, and occlusive diseases affecting the vasculature or other vessels comprising, inter alia, the hepato-biliary and genito-urinary tracts (which are all hereinafter "vessels"). It is known to form such an intraluminal device of a sleeve in which is disposed a plurality of wire stents (see Balko A. et al (1986) Transfemoral Placement of Intraluminal Polyurethane Prosthesis for Abdominal Aortic Aneurysms, 40 Journal of Surgical Research 305-309; Mirich D. et al. (1989) Percutaneously Placed Endovascular Grafts for Aortic Aneurysms: Feasibility Study 170(3) Radiology 1033-1037).

In the past, such devices have commonly been used in the treatment of, or to exclude aneurysms, see United States Letters Patent No's. 5,782,904; 5,968,068; 6,013,092; 6,024,729; 6,045,557; 6,071,307; 6,099,558; 6,106,540 and 6,110,191 each of which is licensed or assigned to and may be available from Edwards Lifesciences LLC (Orange County, California), the instant assignee. WO 99/37242 relates to a renforced aortic graft for use in the treatments of anevrysms. The graft is a flexible tube pre-formed into its in-use shape, the tube including annular reinforements.

Document WO-A-99/15102 describes an intraluminal prosthesis as defined in the preamble of claim 1.

Whatever the purpose for which an intraluminal device is being used, it has the capacity to be inserted percutaneously through a distal (or proximal) and connecting vessel to that in which the device is to be used, for example, through the femoral artery in a catheter, where the device is intended to be used in the treatment of a lesion within the aorta. Upon release of the device from the catheter it may expand to a desirable size, and may extend above and below the lesion thereby bridging the lesion. This method of inserting the device into the body of a patient is applicable where the invention is used in the treatment of aneurysmal disease or stenotic disease.

There are several potential problems associated with most known intraluminal devices. For instance, conventional grafts are not designed to follow the natural curvature of some vessels and may, therefore, kink if required to bridge a section of vessel that has a natural curvature.

Likewise, pursuant to use in particularly tortuous -or specifically diseased vessels - it is often necessary to have "taylor-made" or individually altered/modified grafts on the basis of whether an aortobi-iliac or aorto Uni-iliac emplacement is indicated.

Further to such natural curvature of a vessel, there may also be pathological curvature associated with aneurysmal disease. For example it is known that as an aneurysm situated in, for example, the aorto-iliac region expands, it can cause the artery to deviate in a direction towards the extending aneurysmal sac. This in turn may cause the vessel to shorten in length across this section of artery which may sometimes result in displacement or kinking of any intraluminal device positioned in the artery. Known devices ostensively ignore these types of individuated needs, and have heretofore neither addressed nor ameliorated the majority of the most pressing concerns and issues.

The present invention is directed to an alternative form of intraluminal device which is designed to overcome the above problems, inter alia.

### Summary of the Invention

According to the invention, there is provided an intraluminal device according to claim 1 and an intraluminal device delivery system according to claim 8.

In one preferred embodiment, the tubular body forms an S-shape along its length between the first and at least one second end.

In another embodiment, the intraluminal device is a graft for bridging an aneurysm in an artery of a patient.

The shape of the vessel or vessel portion in which the device is to be disposed may be pre-determined and the device chosen or specifically manufactured such that the shape of the device corresponds with the shape of the vessel or vessel portion. The shape of the vessel or vessel portion may preferably be determined by either ultrasound, plain abdominal films or by CT scanning. In this manner, the device is custom made from imaging of the vessel or vessel portion such that it fits securely within the vessel or vessel portion.

The intraluminal device of the invention comprises a tubular graft body having a length, a first end and at least one second end wherein the first end of the tubular body is angled such that when viewed in a vertical cross-sectional plane, a portion of the tubular body extends outwardly longitudinally a distance greater that the remainder of the first end.

This has the advantage that when the device is disposed in a curved vessel, the first end of the tubular body continues to abut against the wall of the vessel in which the device is disposed even when the vessel deviates from its normal path due to pathological changes in the vessel or if the vessel has a natural curvature. Because the angled first end of the tubular body continues to abut against the surrounding wall of a vessel around substantially its entire periphery it forms a tight seal thereby reducing the likelihood of displacement of the device due to pathological deviation of a vessel from its normal path or due to the natural curvature of a vessel.

Also, there is disclosed herein a method for positioning an intraluminal device according to the invention, including the steps of introducing a catheter or other delivery device into a vein, artery or other vessel in the body of a patient when the device body is in a radially compressed state; causing the intraluminal device to be moved through the catheter or other delivery device until the intraluminal device extends into the vessel from a proximal end of the catheter or other delivery device; causing or allowing the intraluminal device to expand; and withdrawing the catheter or other delivery device along with any other apparatus used to introduce the intraluminal device into the vessel.

The device may be adapted such that it can be brought to a substantially straight configuration along its length and radially compressed to fit internal the catheter or other delivery device. The device is moved through the catheter or other delivery device until it extends from the proximal end of the catheter or other delivery device whereupon the device expands and takes on its pre-determined curved configuration.

The intraluminal device according to this invention may be used in the treatment of aneurysms or stenotic disease. In addition to treating aortic aneurysms the device is particularly suitable for treating aneurysms of the femoral artery, the popliteal artery, the thoracic segment of the aorta, visceral arteries such as the renal and mesenteric arteries, the iliac artery and the sub-clavian artery. Further, in addition to the treatment of stenotic lesions in the peripheral vasculature, the invention may be used in the treatment of, inter alia, vessels comprising the coronary circulation. However the application of the invention for use in the treatment of stenotic disease is not to be understood as limited to the vascular system only, the device may be used to treat stenotic lesions in other vessels including, for example, those comprising the hepato-biliary and genito-urinary tracts.

In cases where the invention is to be used for the treatment of aneurysmal disease, the tubular device body is preferably formed of a thin biocompatible material such as Dacron^{™} or polytetrafluoroethylene (PTFE). The tube material is preferably crimped along its length to increase the flexibility of the device, however, uncrimped material may be used in suitable circumstances. In preferred configurations for use in the treatment of aneurysmal disease, the device body may be formed from a material having a limited amount of diametric elasticity to ensure that it can be expanded into contact with the vessel wall, forming a seal between the wall of the device and the wall of the vessel such that the escape of the vessel contents into the aneurysmal sac is prevented.

Also, there is disclosed herein stent or a series of spaced apart stents which forms a framework to which may be attached an endoluminal graft. The framework of the device body may be circumferentially reinforced along its length by a plurality of separate, spaced-apart, malleable wires. Each of such wires can have a generally closed sinusoidal or zig-zag shape. The wires are preferably formed of stainless steel or another metal or a plastic which is malleable and is biocompatible. If the device is adapted such that it is substantially straight along its length to facilitate packaging within a catheter, the wires may be made from Nitinol^{™} or other such shape memory or heat sensitive material such that when the device is in situ within a vessel, the temperature in the vessel causes the material to take on a pre-determined configuration. This pre-determined configuration of the material causes the device to adopt a pre-determined curved configuration.

Each wire is preferably woven into the fabric of the device body to integrate the body and the reinforcing wires. This prevents any possibility of the wire reinforcement separating from the device body during introduction of the device or throughout its life. If the device body is of a woven material the wires may be interwoven with the device body after its manufacturer. If the device body is not woven but is knitted or of an impervious sheet material then the wires may be threaded through suitable holes formed in the device body. Alternatively the stent or stents may be continuous and may be on the radially inner or the radially outer side of the graft wall. In either case expansion of the graft or grafts will cause the graft to expand and press against the wall of the vessel into which the device has been placed. In one particular configuration, the wires are adapted such that substantially the entire periphery of the angled one end of the tubular body is reinforced.

The tubular graft body may be of the self-expandable type wherein the wires are made from a shape memory or heat sensitive material. In this embodiment, the tubular graft body is ejected from the proximal end of the catheter and into the target vessel. Once in the vessel, the tubular graft body takes on its pre-determined shape. Alternatively, the tubular graft body may be compressed within the lumen of a catheter such that upon release of the tubular graft body from the proximal end of a catheter and into the target vessel, the tubular graft body springs into its pre-determined shape. In a further embodiment, the expansion of the tubular graft body within the target vessel may be aided by way of a balloon which, when inflated pushes the tubular graft body towards the wall of the target vessel.

In addition to or instead of being circumferentially reinforced, the tubular graft body may be longitudinally reinforced. In one embodiment, a longitudinally reinforcing wire may be connected to one or more circumferentially reinforcing wires. The advantage of longitudinal reinforcement is that the tubular graft body is less likely to compress along its length during placement of the tubular graft body in the target vessel, resulting in a concertina affect.

The device is typically substantially of constant diameter along its length, that is, it is substantially cylindrical or may in some instances be frusto-conical in shape with a diameter that increases or decreases along the length of the device.

In another configuration, the device is adapted to bridge an aneurysm that extends up to or slightly beyond an arterial bifurcation. In such a case the device is a graft which has a bifurcation at its downstream end, a so-called "trouser graft", and may be placed wholly within the primary artery. A supplemental graft may then be introduced through subsidiary arteries and overlapped with the lumen of the bifurcated part of the primary graft. In the case of an aneurysm in the aorta, for instance, that extended into each of the common iliac arteries the primary graft would be placed in the aorta. Supplemental grafts which dock with the bifurcated end of the primary graft would then be inserted through each of the common iliac arteries.

### Brief Description of the Drawings

One preferred embodiment of the invention is now described with reference to the accompanying drawings in which:
Figure 1 is a diagrammatic partially cut-away ventral view of a patient with an aortic aneurysm which has been bridged by a device according to the present invention.
Figure 2 is a simplified view of a device according to the prior art.
Figure 3 is a simplified view of a device according to the present invention.
Figure 4 is a detailed longitudinal view of an aortic aneurysm that is bridged by a device according to the prior art.
Figure 5 is a detailed longitudinal view of an aortic aneurysm that is bridged by the device of the present invention.
Figure 6 is a side elevational view of an aortic aneurysm that is bridged by a device according to the prior art.
Figure 7 is a side elevation view of an aortic aneurysm that is bridged by the device of the present invention.
Figures 8a and 8b are representations of a delivery mechanism of one embodiment of the invention.
Figure 9 illustrates an alternate configuration of a graft to be emplaced and implanted according to the teaching of the present invention. That graft does not fall within the scope of the claims.

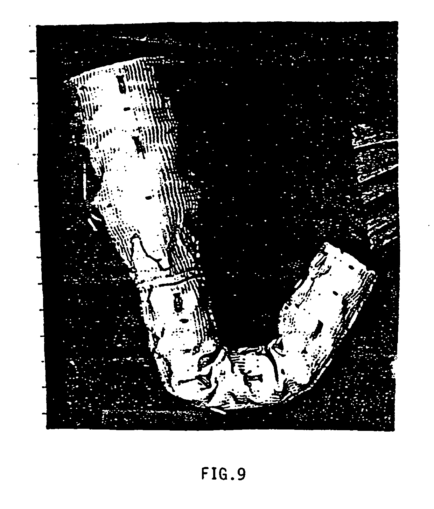

Referring now to Figure 9, an alternate preferred embodiment shows a self-expanding or balloon expandable graft utilizing, for example, a Dacron^{™} graft. According to the instant teachings a graft may be secured to a desired portion of the aorta and iliac arteries by use of the self expanding radial force of wireforms attached to the dacron^{™} graft.

According to this teaching, a graft having at least 28mm of trunk includes a tapered portion. Balloon attachment or self-expansion may be used.

### Best Mode of Carrying Out The Invention

The present inventors have come up with novel ways to enhance the human vasculature by means of grafts which have an alternate, and substantially "pre-formed" shape for certain applications. Unlike known systems, particularly difficult anatomical structures may be ameliorated according to the instant teachings.

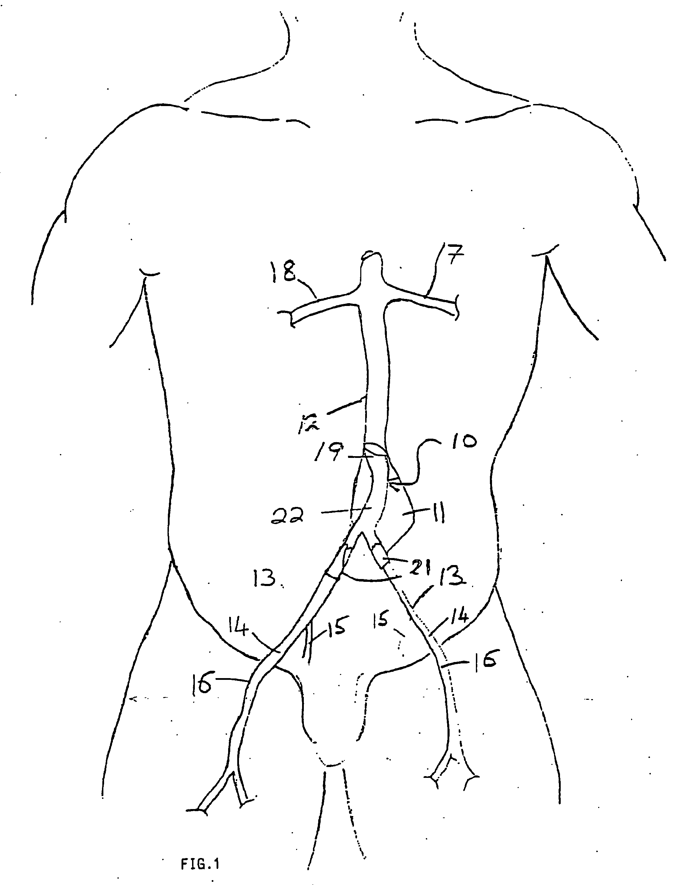

An endovascular graft according to the present invention is generally shown as 10 in the drawings. The endovascular graft 10 is adapted for insertion transfemorally into a patient to achieve bridging and occlusion of an aneurysm 11 present in an aorta 12. It is to be understood that the present invention has a wider applicability and could be utilized in vessels other than the aorta. As is shown in Figure 1 the aorta 12 bifurcates to form the common iliac arteries 13 which subsequently divide into the external 14 and internal 15 iliac arteries, the external iliac artery 14 eventually becoming the femoral artery 16. The aortic aneurysm is located between the renal arteries 17 and 18 and the junctions of the bifurcation of the aorta 12 into the common iliac arteries 13. The graft 10 is inserted inside a catheter 9 and introduced into one of the femoral arteries 16 of a patient Once the catheter 9 is located appropriately with its proximal end in the aorta 12 the graft 10 is ejected from the catheter and expanded so that each end 19 and 21 is in intimated contact around its full periphery with the aorta 12. The graft 10 then bridges the aneurysm 11 and isolates any thrombosis or gelatinous material associated with the aneurysm outside the graft 10 to reduce the risk of embolisation.

The endovascular graft 10 comprises a tube 22 of woven Dacron^{™}. The tube is reinforced along its length with a plurality of separate spaced apart wires that are interwoven in the Dacron^{™}. Between the two ends 19 and 21 the body of the tube 22 curves in a manner that enables the graft 10 to follow the natural or pathological contours of the aorta.

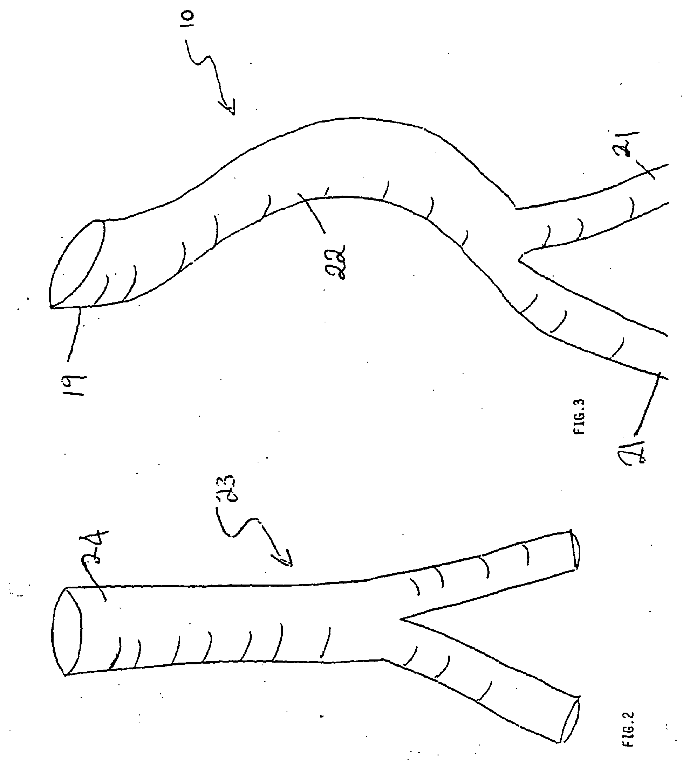

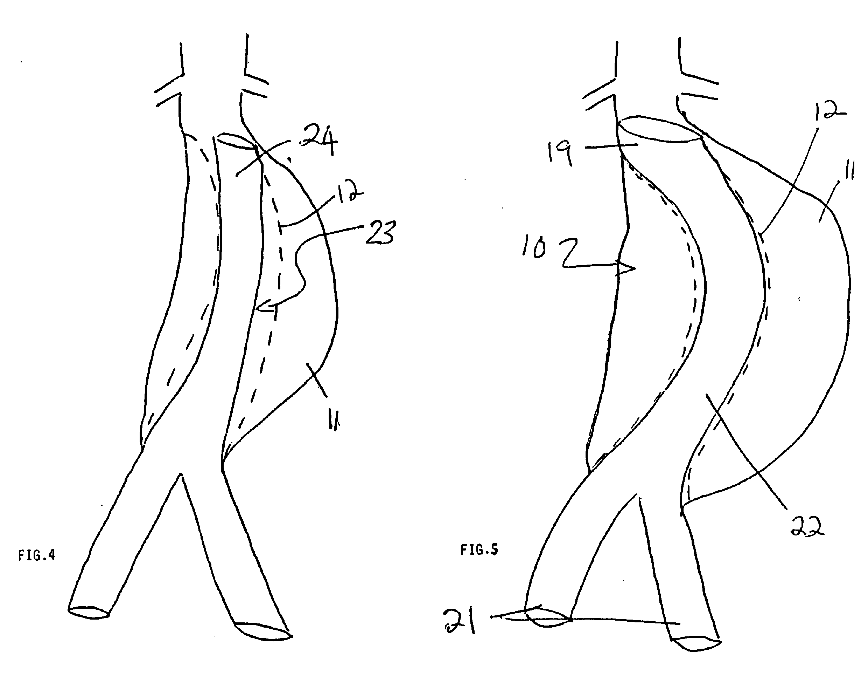

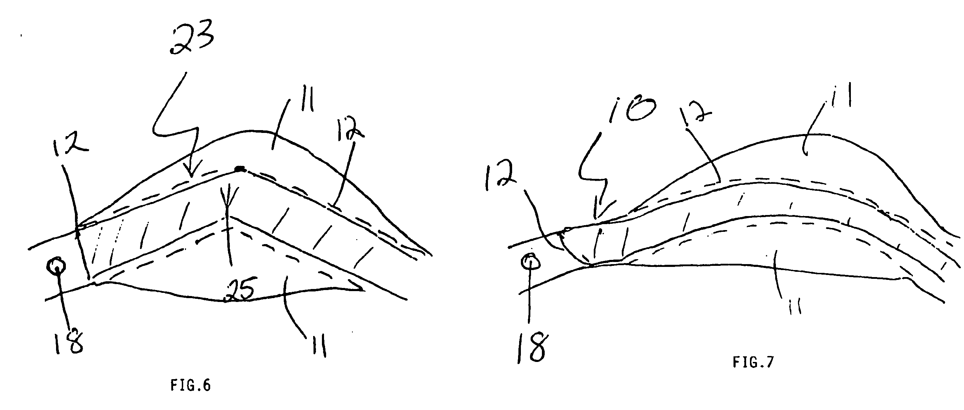

Figures 2 and 3 indicate the difference between the graft of the present invention 10 and conventionally used grafts 23. The conventionally used grafts 23 are substantially straight in design and do not account for either natural curvature of an artery or pathological curvature due to the ballooning and pulling effect of an aneurysm 11. Accordingly, when an aneurysm 11 starts to expand and the aorta 12 is pulled and forced to curve away from its normal path, the grafts of the prior art 23 can become dislodged at end 24 (see Figure 4) or kink at a point 25 along their length, (as depicted in Figure 6).

The benefit of the present invention can be seen in the graft 10 is pre-curved to align with the aorta 12 which is pulled from its natural path due to the ballooning of an aneurysm 11. Further more, end 19 of the graft 10 is angled such that it still abuts against the walls of the aorta 12 when the aorta 12 is curved out by the pull of the aneurysm. In conventional grafts 23, as the aorta 12 is curved, the end 24 of the graft may not fit against the walls of the aorta 12 and the graft can have a tendency to dislodge as a result. This can be seen in Figures 4 and 5 where the section of aorta 12 proximal the renal arteries 17 and 18 deviates towards the expanding aneurysm such that an angle is formed. The angle may in some cases be up to 90° and thus the straight shaped conventional grafts 23 sometimes do not fit securely within the aorta 12, becoming dislodged.

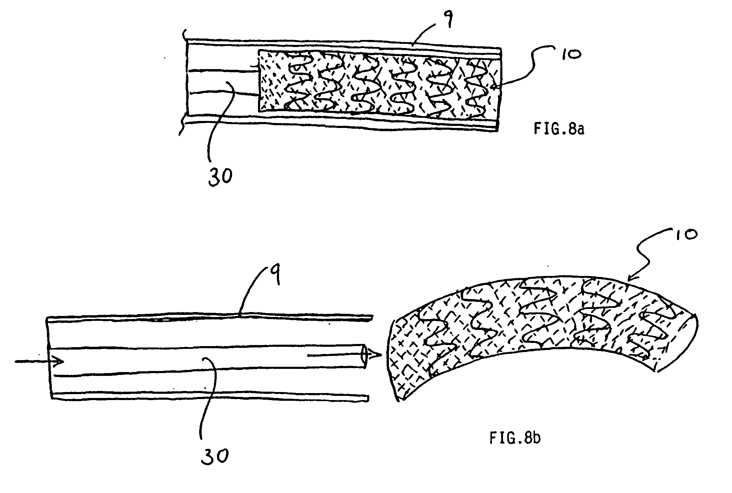

Whilst the graft 10 is adapted to take on a pre-determined configuration such that it aligns with a non-linear vessel, the graft 10 may be inserted into a target vessel in a substantially straight configuration. Figure 8a and 8b depict one means of introducing a graft 10 into a vessel by way of a catheter 9. The graft 10 is forced into a substantially straight configuration within the catheter 9. When positioned correctly within a target vessel, the graft 10 may be ejected from the catheter 9 by way of, for example, a push rod 30 whereupon the graft 10 takes on its pre-determined curved configuration (shown in Figure 8b).

In use, the shape of the vessel in to which the device is to be disposed may be imaged and the device chosen or specifically manufactured such that the shape of the graft 10 corresponds with the shape of the vessel. Imaging may be by way of ultrasound, plain abdominal films or by CT scanning. In this manner, the graft 10 is custom made such that it fits securely within the vessel.

It will be appreciated by persons skilled in the art that numerous variations, and /or modifications may be made to the invention as shown in the specific embodiments without departing from the scope of the claims. The present embodiments are, therefore, to be considered in all respects as illustrative and not restrictive.

## Claims

1. An intraluminal device (10) comprising:
a tubular body (22) having a length, the tubular body having a pre-determined non-linear shape and the tubular body further comprising a first end (19) and at least one second end (21);
wherein the first end is angled such that when viewed in a vertical cross sectional plane, a portion of the tubular body extends outwardly longitudinally a distance greater than the remainder of the first end;
**characterised in that** the predetermined non-linear shape of the tubular body comprises a curvature along its length in both an anterior-posterior plane and a lateral plane and corresponds with a shape of a non-linear shaped portion of a vessel to house the device.

2. An intraluminal device (10) according to Claim 1, where the tubular body (22) further comprises a sigmoid curve disposed along its length between the first (19) and the at least one second end (21).

3. An intraluminal device (10) according to any preceding claim wherein the tubular body (22) is bifurcated at the second end (21) and has two bifurcated limbs.

4. An intraluminal device (10) according to Claim 3 wherein the tubular body (22) further comprises at least one supplemental graft overlapped with one of the bifurcated limbs.

5. An intraluminal device (10) according to any preceding claim wherein the tubular body (22) may be compressed to a first, unexpanded substantially straight shape and expanded to the pre-determined, non-linear shape.

6. An intraluminal device (10) according to any preceding claim wherein the tubular body (22) comprises a tubular body (22) which is circumferentially reinforced along its length by a plurality of separate, spaced apart wires that are interwoven into the tubular body (22).

7. An intraluminal device (10) according to any of the preceding claims wherein the tubular body (22) is longitudinally reinforced by a longitudinally reinforcing wire.

8. An intraluminal device delivery system comprising:
an intraluminal device (10) according to any of claims 1 to 7 ;
and an intraluminal delivery catheter (9) having a length, wherein the catheter is configured such that it is curved along its length in an anterior-posterior plane and a lateral plane.

## Patentansprüche

1. Intraluminales Gerät (10) mit:
einem rohrförmigen Körper (22) mit einer Längserstreckung, wobei der rohrförmige Körper eine vorbestimmte nichtlineare Form besitzt und weiterhin ein erstes Ende (19) und zumindest ein zweites Ende (21) besitzt;
wobei das erste Ende derart abgewinkelt ist, dass sich bei Ansicht in einer vertikalen Querschnittsebene ein Bereich des rohrförmigen Körper auswärts in Längsrichtung mit einem Abstand oder einer Länge erstreckt, der oder die größer ist als der verbleibende Teil von dem ersten Ende,
**dadurch gekennzeichnet, dass** die vorbestimmte nichtlineare Form des rohrförmigen Körpers in Längsrichtung sowohl in einer anterioren- posterioren Ebene als auch in einer lateralen Ebene eine Krümmung oder Kurvenform besitzt und mit der Form eines nichtlinear geformten Bereichs eines Gefäßes zur Aufnahme des Gerätes korrespondiert.

2. Intraluminales Gerät (10) nach Anspruch 1, **dadurch gekennzeichnet, dass** der rohrförmige Körper (22) weiterhin in Längsrichtung zwischen dem ersten Ende (19) und dem zumindest einen zweiten Ende (21) einen S-förmigen Kurvenverlauf aufweist.

3. Intraluminales Gerät (10) nach einem der vorhergehenden Ansprüche, **dadurch gekennzeichnet, dass** der rohrförmige Körper (22) an dem zweiten Ende (21) verzweigt ist und zwei verzweigte Äste aufweist.

4. Intraluminales Gerät (10) nach Anspruch 3, **dadurch gekennzeichnet, dass** der rohrförmige Körper (22) weiterhin zumindest ein zusätzliches Transplantat aufweist, welches einen der verzweigten Äste überlappt oder von diesem überlappt ist.

5. Intraluminales Gerät (10) nach einem der vorhergehenden Ansprüche, **dadurch gekennzeichnet, dass** der rohrförmige Körper (22) zu einer ersten, nicht expandierten substanziell begradigten Form komprimiert werden kann und in die vorbestimmte nichtlineare Form expandiert werden kann.

6. Intraluminales Gerät (10) nach einem der vorhergehenden Ansprüche, **dadurch gekennzeichnet, dass** der rohrförmige Körper (22) einen rohrförmigen Körper (22) aufweist, der in Umfangsrichtung verstärkt ist entlang seiner Länge durch mehrere separate, voneinander beabstandete Drähte, die in den rohrförmigen Körper (22) gewebt oder geschlungen sind.

7. Intraluminales Gerät (10) nach einem der vorhergehenden Ansprüche, **dadurch gekennzeichnet, dass** der rohrförmige Körper (22) in Längsrichtung mittels eines Längsverstärkungsdrahtes verstärkt ist.

8. Überbringungssystem für ein intraluminales Gerät mit:
einem intraluminalen Gerät (10) nach einem der Ansprüche 1 bis 7;
einem intraluminalen Überbringungskatheter (9) mit einer Länge, wobei der Katheter derart konfiguriert ist, dass dieser entlang seiner Länge in einer anterioren- posterioren Ebene und einer lateralen Ebene kurvenförmig ist oder gekrümmt ist.

## Revendications

1. Dispositif intraluminal (10) comprenant :
un corps tubulaire (22) ayant une longueur, le corps tubulaire ayant une forme non linéaire prédéterminée et le corps tubulaire comprenant en outre une première extrémité (19) et au moins une seconde extrémité (21) ;
dans lequel la première extrémité est coudée de sorte que lorsqu'elle est observée dans un plan transversal vertical, une partie du corps tubulaire s'étend longitudinalement vers l'extérieur sur une distance supérieure au reste de la première extrémité ;
**caractérisé en ce que** la forme non linaire prédéterminée du corps tubulaire comprend une courbure le long de sa longueur à la fois dans un plan antérieur-postérieur et un plan latéral, et correspond à une forme d'une partie formée de manière non linéaire d'un vaisseau pour loger le dispositif.

2. Dispositif intraluminal (10) selon la revendication 1, dans lequel le corps tubulaire (22) comprend en outre une courbe sigmoïde disposée le long de sa longueur entre la première (19) et la au moins une seconde extrémité (21).

3. Dispositif intraluminal (10) selon l'une quelconque des revendications précédentes, dans lequel le corps tubulaire (22) est bifurqué au niveau de la seconde extrémité (21) et a deux membres bifurqués.

4. Dispositif intraluminal (10) selon la revendication 3, dans lequel le corps tubulaire (22) comprend en outre au moins une greffe supplémentaire chevauchée avec l'un des membres bifurqués.

5. Dispositif intraluminal (10) selon l'une quelconque des revendications précédentes, dans lequel le corps tubulaire (22) peut être comprimé selon une première forme sensiblement droite non expansée et expansé selon la forme non linéaire prédéterminée.

6. Dispositif intraluminal (10) selon l'une quelconque des revendications précédentes, dans lequel le corps tubulaire (22) comprend un corps tubulaire (22) qui est renforcé de manière circonférentielle le long de sa longueur par une pluralité de fils séparés, espacés, qui sont entrelacés dans le corps tubulaire (22).

7. Dispositif intraluminal (10) selon l'une quelconque des revendications précédentes, dans lequel le corps tubulaire (22) est renforcé longitudinalement par un fil de renforcement longitudinal.

8. Système de pose de dispositif intraluminal comprenant:
un dispositif intraluminal (10) selon l'une quelconque des revendications 1 à 7 ; et
un cathéter de pose intraluminal (9) ayant une longueur, dans lequel le cathéter est configuré de sorte qu'il est incurvé le long de sa longueur dans un plan antérieur-postérieur et un plan latéral.
